# EUROPEAN PATENT SPECIFICATION

(11) **EP 0 841 896 B1**
(45) Date of publication and mention of the grant of the patent: **13.11.2002**
(21) Application number: 96925440.8
(22) Date of filing: 24.07.1996
(51) Int. Cl.: A61K 7/48, A61K 9/06, A61K 9/107

(54) **REVERSE GELS COMPRISING A CONTINUOUS FLUORINATED PHASE**
UMKEHRGELE MIT EINER KONTINUIERLICHEN FLUORIERTEN PHASE
GELS INVERSES COMPRENANT UNE PHASE FLUOREE CONTINUE

(30) Priority: 24.07.1995 FR 9508965
(43) Date of publication of application: 20.05.1998
(73) Proprietor: ALLIANCE PHARMACEUTICAL CORPORATION, San Diego California 92121 (US)
(72) Inventor: KRAFFT, Marie-Pierre, F-06100 Nice (FR); RIESS, Jean, G., F-06950 Falicon (FR)
(74) Representative: Van Malderen, Joelle
(86) International application number: US9612138
(87) International publication number: WO97003644

(56) References cited:
- WO-A-95/09606
- WO-A-95/33447
- FR-A- 2 679 150

## Description

The present invention is directed to fluorocarbon gels for the transport of pharmaceutical compounds and other active principles.

### Background of the Invention

Highly fluorinated or perfluorinated compounds are known for being chemically and biologically inert as well as their ability to dissolve gases, including the respiratory gases (Riess J.G. in Fluorine in Medicine, R.E. Banks, K.C. Lowe (eds), Rapra Technol. Ltd., Shawbury, U.K. 1994). Due to this gas-carrying ability, fluorocarbons have proved to be useful in the biomedical field in a pure form or as components of emulsions. In the form of direct emulsions (oil-in-water), fluorocarbons can be used as temporary blood substitutes in perioperative hemodilution, treatment of cardiac or cerebral ischemia, as adjuvant in the treatment of cancer, or as oxygenating fluid for the preservation of organs. (Riess J.G. in "Fluorocarbon-based in vivo oxygen Transport and Delivery Systems), Vox Sanguinis, 61, 225 (1991). In pure or "neat" form, fluorocarbons are presently being tested clinically for the treatment of the respiratory distress syndrome (Liquivent®, Alliance Pharm. Corp. San Diego, USA, T.H. Shaffer and col. in ISBS, Biomat., Art. Cells Immob. Biotech., 24, 1994, and in Pediatric Pulmonology, 14, 102 (1992)).

Fluorocarbon emulsions for the transport of oxygen and of active principles in cosmetics and dermatology have been described in WO 94/00098. At low concentrations (about 0.1 to 50% v/v) some of these emulsions may be incorporated in relatively low viscosity gels.

The use of fluorocarbons in the form of emulsions or of direct gels i.e. in which the continuous phase is water, has also been described. These formulations of fluorocarbon gels require the use of a gelifying agent and a stabilizing agent. Thus, US Pat. No. 5,073,378 describes direct gels for use in the treatment of burns. The disclosed gels, wherein the continuous phase is water, comprise solutions of collagen containing a growth factor and a small quantity of fluorocarbon.

US Pat. No. 4,569,784 describes a stable fluorocarbon gel comprising, at most, 50% by volume of fluorocarbon. A considerable quantity of surfactant must be incorporated in the formulations in order to stabilize the resulting gel. The disclosed gels are prepared by a complex procedure involving a step in which an emulsion is concentrated by centrifugation.

The document FR-A-2 630 347 describes direct gels of fluorocarbons comprising a very high proportion of water (60 to 98% in volume). As a consequence the quantity of fluorocarbon is low.

Other direct gels comprising an aqueous continuous phase and highly concentrated in fluorocarbon are known. Examples include the gels described in the Patent application FR-A-2 710 840. These gels are formed of polyaphrons of fluorocarbon surrounded by the aqueous continuous phase and, accordingly, could not permit the controlled release of hydrosoluble active principles.

The document FR-A-2679150 describes a preparation comprising fluorocarbon and a highly fluorinated compound and a lipophilic organic compound used as a stabilizing agent.

The document WO95/09606 describes viscoelastic compositions of fluorinated organic compounds used as thickeners or as stabilizing agents.

Direct gels were also disclosed in Applicants' co-pending U.S. patent application 08/483,946. This application discloses the use of lipophilic/fluorophilic compounds to form stable dispersions which may advantageously be used for drug delivery.

Reverse gels, those with an aqueous discontinuous phase, formed from phospholipids and from a hydrogenated oil, such as the isopropyl palmitate, have been described by Scartazzini et al (J. Phys. Chem. 1988, 92, 829). These reverse gels (or organogels) were studied by Willimann et al (J. Pharm. Sci., 1992, 81, 871) as matrixes for the transdermal transport of medicaments (scopolamine, broxaterol), of aminated acids and of peptides, and can also be used as medium for enzymatic reactions. However, these reverse gels do not contain any fluorocarbons.

Reverse gels containing fluorocarbon as the continuous phase and water as the discontinuous or internal phase have been described in the literature (Ravey JC et al, Prog. colloid polym. Sci., Vol. 82, p. 218-28, 1990) but these gels are limited to a maximum fluorocarbon concentration of 50% v/v. Moreover, the disclosed gels contain a fluorinated surfactant of the polyoxyethylene type.

Conversely, there does not appear to be any mention of the use of fluorocarbon reverse gels comprising phospholipids or other nonfluorinated surfactants. The very low solubility of such surfactants in fluorocarbons would tend to lead those skilled in the art to believe that they could not be used to form stabilized gel systems comprising a fluorocarbon continuous phase.

Accordingly, there remains a need for reverse gels comprising high concentrations of at least one fluorinated compound and a biocompatible surfactant wherein the gels are capable of dissolving and transporting large amounts of oxygen and/or active principles. In such compositions the oxygen would primarily be carried in the fluorocarbon continuous phase while the active principle or pharmaceutical compound would preferably be carried in the aqueous dispersed phase although it may be distributed among the two phases.

There also remains a need to have a well defined gel transport system capable of carrying both hydrophilic and lipophilic active principles and pharmaceutical compounds.

### Summary of the Invention

These and other objects are achieved by the gel compositions and methods of the present invention which provide reverse fluorochemical gels capable of carrying physiologically significant amounts of oxygen and hydrophilic or lipophilic active principles. Surprisingly it was found that such reverse gels could be formed by combining relatively high concentrations of fluorocarbon with nonfluorinated surfactants such as phospholipids. Through the use of partially hydrogenated and partially fluorinated ("HC/FC") compounds the nonfluorinated surfactants act to form generally homogeneous dispersions. This novel use of such surfactants unexpectedly provides for the formation of stable gel systems having effective transport and delivery capabilities for physiological gases and active principles such as pharmaceutical compounds.

In a broad aspect the fluorinated reverse gels disclosed herein comprise an aqueous phase dispersed in a continuous fluorinated phase. Preferably, the continuous fluorinated phase comprises a mixture of at least one highly fluorinated organic compound and at least one (HC/FC) organic compound. An effective dispersing amount of at least one nonfluorinated surfactant maintains the stability of the gel. Selected embodiments of the invention may further comprise additives, including active principles, incorporated within the fluorinated reverse gel. Additives may be distributed in the disperse aqueous phase, the continuous fluorinated phase, the interface between these two phases or dispersed throughout the entire gel.

Accordingly, one aspect of the present invention comprises fluorinated reverse gels comprising:
a continuous phase comprising at least one highly fluorinated organic compound and at least one partially fluorinated and partially hydrogenated (HC/FC) organic compound;
a dispersed aqueous phase; and
an effective dispersing amount of at least one nonfluorinated surfactant wherein said dispersed aqueous phase comprises less than about 50% v/v of said reverse gel.
In the gels of the present invention the concentration of the continuous fluorinated phase may comprise from about 50% v/v to about 99.9% v/v, depending on the characteristics desired. Preferably, the concentration of the continuous phase will comprise more than about 60% v/v and even more preferably greater than 70% v/v. For many applications continuous phase concentrations of greater than about 75% v/v or about 80% v/v will be desired. For other applications, particularly those that are topical in nature, gels having continuous phase concentrations greater than about 85% v/v or about 90% v/v will be preferred. Within the continuous phase the concentration of the highly fluorinated organic compound may vary from about 0.01% v/v to about 99.9% v/v, depending on the properties desired.

It will, therefore, be appreciated that the viscosity of the instantly disclosed fluorinated reverse gels may be selected to enhance the uptake of any incorporated active principles or prolong retention at the site of application. Gel components and concentrations may be altered to provide a composition having the desired viscosity. For example, the final concentration of the disperse aqueous phase may be increased to yield a relatively less viscous gel. Similarly, altering the procedure used to form the gel, i.e. varying the power or duration of sonication, can produce dispersions of different consistencies. However, in each case the gels of the present invention exhibit a remarkable stability due to the advantageous properties of the nonfluorinated surfactants.

Thus, other embodiments of the present invention include methods for forming a fluorinated reverse gel comprising the steps of:
combining at least one partially fluorinated and partially hydrogenated (HC/FC) organic compound with an effective dispersing amount of at least one nonfluorinated surfactant and at least one highly fluorinated organic compound to provide a dispersion preparation; and
admixing said dispersion preparation with an aqueous solution to provide a fluorinated reverse gel comprising a disperse aqueous phase of less than about 50% v/v.

Those skilled in the art will appreciate that different techniques may be employed within the broad scope of the present invention to actually form the desired reverse gel. For example, the admixing or dispersing step may be accomplished through sonication, mechanical convection or other conventional means. Similarly, the order in which the components are combined is not critical and may be adjusted for ease of production or cost considerations.

In this respect a particularly preferred embodiment of the present invention is directed to methods for the preparation of a fluorinated reverse gels comprising the steps of:
combining a nonfluorinated surfactant with at least one partially fluorinated and partially hydrogenated (HC/FC) organic compound to obtain a homogeneous mixture;
dispersing said homogeneous mixture in at least one highly fluorinated organic compound to provide a dispersion preparation; and
adding said dispersion preparation to an aqueous solution to provide a fluorinated reverse gel comprising a dispersed aqueous phase of less than about 50% v\v.

It will be appreciated that the gels of the present invention may be used in combination with various additives, including active principles, or by themselves. Preferably, the reverse gels of the present invention are used in connection with the biomedical field, in particular for transdermal transport of active principles such as therapeutic or diagnostic agents. In other cases the gels may be used, with or without additives, as protective creams, for lubrication, in cosmetics, or to create a deposit effect, for example subcutaneous or in a cavity of the body. Particularly preferred routes of administration include ocular, oral, topical, nasal vaginal and rectal as well as parenteral.

Accordingly, in another aspect the present invention provides a method for delivering an active principle to a patient comprising:
providing fluorinated reverse gel comprising a disperse aqueous phase; a continuous fluorocarbon phase comprising at least one partially fluorinated and partially hydrogenated (HC/FC) organic compound and at least one highly fluorinated organic compound; an effective dispersing amount of at least one nonfluorinated surfactant; at least one active principle; and administering said fluorinated reverse gel to a patient. Other objects, features and advantages of the present invention will be apparent to those skilled in the art from a consideration of the following detailed description of preferred exemplary embodiments thereof.

### Detailed Description of the Invention

While the present invention may be embodied in many different forms, disclosed herein are specific illustrative embodiments thereof that exemplify the principles of the invention.

The disclosed invention comprises reverse gels of the water-in-fluorocarbon type. These gels tend to comprise aqueous microdomains dispersed in a relatively large proportion of fluorocarbon and, in preferred embodiments, may be used for the transport and delivery of therapeutic or diagnostic agents. The continuous phase composed of fluorocarbon is characterized by its ability to dissolve physiological gases such as nitric oxide or oxygen, its generally soothing effect on tissue and its relative impermeability towards non-fluorinated substances.

These properties give the reverse gels of the present invention the important ability to diminish the rate of release of encapsulated hydrophilic drugs. Accordingly, the reverse gels of the present invention are particularly advantageous for the delivery of medicaments by the transdermal route. They may also be used to protect the epidermis and enhance the treatment of wounds and burns. In this respect they may be used to reduce the risk of the developing bedsores. In addition, the disclosed gels are also suitable for subcutaneous use and as a drug depot in the natural cavities of the body where they provide for deposit and of progressive release of an active principle.

As previously indicated, the water-in-fluorocarbon reverse gels are relatively stable over time and can be sterilized thermally.

In a broad aspect the present invention provides fluorinated reverse gels comprising:
a continuous phase comprising at least one highly fluorinated organic compound and at least one partially fluorinated and partially hydrogenated (HC/FC) organic compound;
a dispersed aqueous phase; and
an effective dispersing amount of at least one nonfluorinated surfactant wherein said dispersed aqueous phase comprises less than about 50% v/v of said reverse gel.
As used herein, the term gel indicates a preparation having a viscosity greater than the respective viscosities of the individual fluorocarbon and water phases. The term reverse gel designates a viscoelastic and homogeneous dispersion of a minor aqueous phase in a fluorinated continuous phase.

The reverse character of these gels, i.e. the presence of a continuous fluorinated phase, is demonstrated by the fact that it is easy to dilute them in a fluorocarbon and not in an aqueous solution.

According to the invention, the gel can also comprise one or more additives chosen from among cosurfactants, active principles, mineral salts, amino acids, buffering agents, oncotic agents, osmotic agents, organic solvents, physiological gases, dispersing agents, polymers, absorption enhancers (agents susceptible of augmenting percutaneous absorption), immunoactive principles, nutritive agents, stabilizers, and other compounds capable of improving the characteristics, stability, efficacy and tolerance of the reverse gels. As used herein, the term "active principles" is held to mean any bioactive agent, including therapeutic or diagnostic agent, that may be used to treat a condition or disease. Those skilled in the art will appreciate that these additives may be dispersed throughout the aqueous phase, the fluorinated continuous phase, in each of the two phases or in the surfactant film.

Thus, the aqueous phase can contain one or several hydrosoluble additives and, in particular, active principles. Conversely, the fluorinated continuous phase may be used to transport, in a dissolved or dispersed form, a relatively hydrophobic or fluorophilic active principle. The surfactant film between the phases can also contribute to the transport of active principles.

Solvents and other organic derivatives such as, for example, ethanol, N-methylpyrrolidone, dimethylsulfoxide or methyldecylsulfoxide, or laurocapranne, can be added to facilitate the penetration of the active principles into the dermis. In preferred embodiments these absorption enhancers are miscible with the aqueous phase and are dispersed in conjunction with it when the gels are formed. Such absorption enhancers can markedly increase the uptake of therapeutic or diagnostic compounds thereby lowering the effective dose.

The highly fluorinated organic compounds comprising a portion of the fluorinated continuous phase in the disclosed preparations are typically chosen for their low toxicity, gas dissolving capacity, surface tension and spreading coefficient. Particularly preferred fluorochemicals will be capable of delivering therapeutically significant amounts of gases including nitric oxide or oxygen. Such fluorochemicals may make up to 99.9% v/v of the fluorinated continuous phase.

In general, the highly fluorinated or perfluorinated organic compounds may be linear, branched or cyclic, saturated or unsaturated fluorinated compounds. Conventional structural derivatives of these fluorochemicals are also contemplated as being within the scope of the present invention as well. In addition, these totally or partially fluorinated compounds may contain one or more hetero-atoms such as O, S or N and/or atoms of bromine or chlorine. As used herein the term "highly fluorinated" indicates that at least 30% of the hydrogen atoms in the hydrocarbon oil or derivative thereof have been replaced with fluorine atoms. Preferably, these fluorochemicals comprise from 2 to 16 carbon atoms and include, but are not limited to, linear, cyclic or polycyclic perfluoroalkanes, bis(perfluoroalkyl)alkenes, perfluoroethers, perfluoroamines, perfluoroalkyl bromides, perfluoroalkyl chlorides and mixtures thereof.

In particularly preferred embodiments the fluorocarbon is preferably perfluorooctyl bromide, perfluorooctylethane, the perfluoropolyethers fabricated by Ausimont (Centre Recerche e Sviluppa, BOLLATE, Italy) and APF-brand fluorocarbons fabricated by Air Products and Chemicals, Inc. (Allentown, PA).

Other preferred fluorochemicals include perfluoroctane 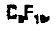 perfluorodecane C₁₀F₂₂, perfluorodecyl bromide C₁₀F₂₁Br (PFDB), bis(perfluorobutyl)ethene (F-44E) or perfluorodecalin (FDC). In addition to the aforementioned compounds, exemplary fluorochemicals which are contemplated for use in the present invention generally include halogenated fluorochemicals (i.e. CₙF₂ₙ₊₁X, XCₙF₂ₙX, where n = 2-10, X = Br, Cl or I) and in particular, 1-bromo-F-butane n-C₄F₉Br, 1-bromo-F-hexane (n-C₆F₁₃Br), 1-bromo-F-heptane(n-C₇F₁₅Br), 1,4-dibromo-F-butane and 1,6-dibromo-F-hexane. Other useful brominated fluorochemicals are disclosed in US Patent No. 3,975,512 to Long and are incorporated herein by reference. Specific fluorochemicals having chloride substituents, such as perfluorooctyl chloride (n-C₈F₁₇Cl), 1,8-dichloro-F-octane (n-ClC₉F₁₉Cl), 1,6-dichloro-F-hexane (n-ClC₆F₁₂Cl), and 1,4-dichloro-F-butane (n-ClC₄F₉Cl) are also preferred.

Still other useful fluorinated compounds comprise, for example, perfluorodiisopropyldecalin, perfluoronbutyldecalin, perfluorodixylylmethane, perfluorodixylylethane, perfluoroperhydrophenanthrene and their mixtures.

In addition to the highly fluorinated organic compounds the fluorinated phase of the reverse gels of the present invention comprises at least one partially fluorinated and partially hydrogenated (HC/FC) organic compound. While not wishing to limit the present invention in any way or be bound to any one theory, it is believed that these HC/FC compounds interact with the nonfluorinated surfactants facilitating an isotropic dispersion and providing for the novel characteristics of the disclosed gels.

In preferred embodiments the partially fluorinated/partially hydrogenated (FC/HC) compounds used correspond to one of the following formulae: CₙF₂ₙ₊₁CₘH₂ₘ₊₁, CₙF₂ₙ₊₁CH=CHCₘH₂ₘ₊₁ and CₙF₂ₙ₊₁CH₂CH=CHCₘH₂ₘ₊₁ where 2≤n≤12 and 1≤m≤16. In particularly preferred embodiments the FC/HC compound will comprise C₆F₁₃C₁₀H₂₁ (Famblin Z from Ausimont).

Preferably, the reverse emulsion contains between 0.01% and 10% w/v of a nonfluorinated surfactant or mixture of surfactants. Because of their excellent biocharacteristics, phospholipids are generally the most preferred class of hydrogenated surfactants. More particularly, phospholipids that tend to adopt the reverse hexagonal phase at low temperatures and concentrations are favored. Accordingly, phosphatidylethanolamines and phosphatidic acids and the like are preferred. In particularly preferred embodiments, the phospholipid has some molecular solubility in the continuous oily phase. Selected embodiments of the invention comprise, phosphatidic acids or phosphatidylethanolamines containing at least one mono-unsaturated fatty acyl moiety. Most preferably, the phosphatidic acid or phosphatidylethanolamine is 1,2-dioleoylphosphatidic or 1,2-dioleoylphosphatidylethanolamine respectively.

Other nonfluorinated surfactants suitable for use in the emulsions of the present invention include, but are not limited to, phosphatidylcholines, N-monomethyl-phosphatidylethanolamines, N,N-dimethyl-phosphatidyl-ethanolamines, phosphatidyl ethylene glycols, phosphatidylmethanols, phosphatidylethanols, phosphatidylpropanols, phosphatidylbutanols, phosphatidylthioethanols, diphytanoyl phosphatides, cardiolipins, cholesterol, glyceroglycolipids, egg yolk phospholipids, salts of fatty acids, phosphatidylserines, phosphatidylglycerols, aminoethylphosphonolipids, dipalmitoyl phosphatidylcholesterol, ether-linked lipids and dicetylphosphate.

Conventional detergents with low hydrophilic-lipophilic balance (ca. 2-10) may also be used as surfactants. Preferred detergents of this type include SPANS® (sorbitan tetraoleate, sorbitan tetrastearate, sorbitan tristearate, sorbitan tripalmitate, sorbitan trioleate, and sorbitan distearate). Guerbet alcohol ethoxylates, dialkyl noni surfactants and dialkylzwitterionic surfactants, including betaines and sulfobetaines, are also contemplated for use as emulsifying agents.

In particularly preferred embodiments, the nonfluorinated surfactants used in the invention are phospholipids. Natural, synthetic or modified phospholipids are used, for example natural or modified egg yolk lecithin.

In addition to the principle nonfluorinated surfactant, one or more secondary surfactants, or co-surfactants, can be added if necessary.

The reverse gels of the invention may be obtained by the dispersion, under continuous stirring, of the nonfluorinated surfactant in the FC/HC compound to obtain a homogeneous mixture. This mixture may then dispersed in the highly fluorinated organic compound, either by mechanical stirring or by sonication to provide a dispersion preparation. The addition of water to this dispersion with mixing leads to the formation of the reverse gel. The gels obtained are more or less transparent according to the formulation.

Accordingly, as previously alluded to the gels may be prepared through the steps of:
combining a nonfluorinated surfactant with at least one partially fluorinated and partially hydrogenated (HC/FC) organic compound to obtain a homogeneous mixture;
dispersing said homogeneous mixture in at least one highly fluorinated organic compound to provide a dispersion preparation; and
adding said dispersion preparation to an aqueous solution to provide a fluorinated reverse gel comprising a dispersed aqueous phase of less than about 50% v\v.

The steps may be accompanied by conventional techniques such as sonication, mechanical mixing or stirring to ensure homogeneous dispersion of the components and an isotropic gel matrix. Based on the formulation used the gels may be transparent or opalescent.

When the composition is to be used in biomedical applications, the reverse gel can further be subjected to a sterilization treatment, which can be effected for example by heating in an autoclave.

As alluded to previously, when the gel comprises additives, including active principles, these can be added to the aqueous phase, to the oily phase or to both phases. This may be effected during preparation of the gel by combining lipophilic additives with the FC/HC compound and the hydrophilic additives to the aqueous solution that will form the aqueous phase. Conversely, the desired active principle may be incorporated following formation of the reverse gel. Regardless of when they are added the reverse gels of the present invention are capable of delivering additives to a patient, including active principles such as therapeutic or diagnostic agents. Those skilled in the art will appreciate that the desired compounds may be incorporated in either the disperse phase, the continuous phase or at the interface between the phases. The ultimate disposition of the additive will primarily depend on its molecular properties and the technique used to form the reverse gel incorporating the compound. For example, lipophilic agents may be combined with the fluorinated continuous phase at any time including prior to, during or subsequent to the formation of the gel. Conversely, any water soluble agent may be combined with the discontinuous phase used to form the selected reverse gel.

As used herein, the term active principle is defined to mean any bioactive agent, pharmaceutical compound or composition, including diagnostic and therapeutic agents, which may be administered to an animal to treat a disease, disorder or condition. Preferred active principles include hydrophilic drugs with solubility in water and lipophilic drugs.

Preferably, the formulations of the present invention incorporate less than about 50% w/v of an active principle. Diagnostic agents will typically be incorporated at higher concentrations than therapeutic agents. The precise amount of active principle incorporated in the reverse gels of the present invention is dependent upon the agent of choice, the required dose, and the form of the drug actually used for incorporation. Those skilled in the art will appreciate that such determinations may be made by using well-known pharmacological techniques in combination with the teachings herein.

Preferred active principles comprise hydrophilic and lipophilic respiratory agents, antibiotics, antivirals, mydriatics, antiglaucomas, anti-inflammatories, antihistaminics, antineoplastics, anesthetics, opthalmic agents, enzymes, cardiovascular agents, nucleic acids, genetic material, viral vectors, immunoactive agents, imaging agents, immunosuppressive agents, peptides, proteins, physiological gases, gastrointestinal agents and combinations thereof.

Further exemplary embodiments of the present invention comprise anti-inflammatory agents such as the glucocorticosteroids (i.e. cortisone, prednisone, prednisolone, dexamethasone, betamethasone, Beclomethasone diproprionate, Triamcinolone acetonide, Flunisolide), xanthines (i.e. theophylline, caffeine), chemotherapeutics (i.e. cyclophosphamide, lomustine, methotrexate, cisplatin, carboxy platin, taxane derivatives), antibiotics (i.e. aminoglycosides, penicillins, cephalosporins, macolides, quinolones, tetracyclines, chloramphenicol), bronchodilators such as the B₂-agonists (i.e. adrenaline, isoprenaline, salmeterol, salbutamol, terbutaline, formoterol) and surfactants. Still other exemplary embodiments include α/β adrenergic blockers (i.e. Normodyne, Trandatel, angiotensin converting enzyme inhibitors (i.e. Vasotet), antiarrhythmics, beta blockers, calcium channel blockers, inotropic agents, vasodilators, vasopressors, anesthetics (i.e. morphine) and ophthalmic agents (i.e. Polymyzin B, Neomycin, Gramicidin). In accordance with the present invention, those skilled in the art will appreciate that various forms of these compounds may be used to obtain the desired therapeutic index.

Because the reverse emulsions of the present invention are uniquely suited far use in a wide variety of physiological applications such as ocular, topical, oral, pulmonary, rectal, synovial, subcutaneous, intraperitoneal, nasal, vaginal, or aural administration of medicaments or diagnostic compounds, almost any compound may be effectively delivered. Accordingly, the foregoing list of active principles is exemplary only and not intended to be limiting. It will also be appreciated by those skilled in the art that the proper amount compound administered and the timing of the dosages may be determined for the formulations in accordance with already-existing information and without undue experimentation.

Delivery of active principles by the transdermal route, as an alternative to the more traditional ways of administration, i.e. orally or by percutaneous injection, is particularly preferred. Other preferred delivery routes comprise the formation of a deposit of active substances, for example under the epidermis, the dermis or in one of the cavities of the body. Particularly preferred active principles generally belong to one of the following families: antiinflammatories, antibiotics, antifungals, antibacterials and antineoplasics.

### Example 1

Reverse water-in-fluorocarbon gel containing perfluorooctyl bromide (perflubron), a FC/HC compound (C6F13C10H21, F6H10), water and a phospholipid.

The phospholipid (of egg-yolk, Lipoid) (100 mg) is dispersed in the mixed fluorinated/hydrocarbon derivative C₆F₁₃C₁₀H₂₁ (4 ml) by sonication (5 min, 30°C). The perfluorooctyl bromide (6 ml) is added slowly. A fluid opalescent dispersion is obtained. The purified water (0.18 ml) is added dropwise to this dispersion. A yellow, transparent, continuous-phase fluorocarbon is obtained in a few seconds after the addition of water. This gel can be sterilized thermally (121°C, 15 min, 15 N m-2) without apparent degradation. No apparent degradation (crystallization or phase-separation) occurs after 6 months at room temperature.

### Examples 2 to 9

The same operational procedure as in Example 1 is followed to prepare the gels of the attached table. Different phospholipids, different fluorocarbons and/or quantities of fluorocarbon are used in addition to water. In all cases stable gels are obtained.

**Table 1**

| Ex | Fluorocarbon (mL) | Water (mL) | Phospholipid (mg) | Gel Characteratics |
|---|---|---|---|---|
| 2 | PFOBa/F6H10b 6/4 | 0.18 | Hydrogenated Lipoid E 100-3 100 mg | Transparent |
| 3 | PFPEc:F6H10 6/4 | 0.18 | Lipoid, Egg yolk 100 mg | yellow opalescent |
| 4 | APF-240d/F6H10 6/4 | 0.18 | Lipoid, Egg yolk 100 mg | yellow opalescent |
| 5 | PFPE/F6H10 6/4 | 0.18 | Lipoid, Egg yolk 100 mg | yellow opalescent |
| 6 | PFOB/F6H10 6/4 | 0.05 | Lipoid, Egg yolk 100 mg | yellow opalescent |
| 7 | PFOB/F6H10 6/4 | 0.025 | Lipoid, Egg yolk 100 mg | yellow opalescent |
| 8 | PFOB/F6H10 6/4 | 0.54 | Lipoid, Egg yolk 100 mg | yellow opalescent |
| 9 | PFOB/F6H10 6/4 | 0.9 | Lipoid, Egg yolk 500 mg | yellow opalescent |
| a) PFOB : perfluorooctyl bromide | | | | |
| b) F6H10 : C₆F₁₃C₁₀H₂₁ (Fomblin Z from Ausimont) | | | | |
| c) PFPE : perfluoropolyether, | | | | |
| d) APF 240: (perfluorodiisopropyldecalin commercialized by Air Products and Chemicals Inc.)] | | | | |

The foregoing examples serve to demonstrate the stability and versatility of the present invention. In particular, sterilization of the reverse gel did not adversely affect the consistency or stability of the preparation.

## Claims

1. A fluorinated reverse gel comprising :
a continuous phase comprising at least one highly fluorinated organic compound wherein at least 30% of the hydrogen atoms in the corresponding hydrocarbon oil or derivative thereof have been replaced with fluorine atoms and at least one partially fluorinated and partially hydrogenated (HC/FC) organic compound;
a dispersed aqueous phase; and
an effective dispersing amount of at least one nonfluorinated surfactant wherein said dispersed aqueous phase comprises less than 50% v/v of said reverse gel.

2. The reverse gel of Claim 1, wherein said at least one nonfluorinated surfactant is selected from the group consisting of alcohols, salts of fatty acids, phosphatidylcholines, N-monomethyl-phosphatidylethanolamines, phosphatidic acids, phosphatidyl ethanolamines, N,N-dimethyl-phosphatidyl-ethanolamines, phosphatidyl ethylene glycols, phosphatidylmethanols, phosphatidylethanols, phosphatidylpropanols, phosphatidylbutanols, phosphatidylthioethanols, diphytanoyl phosphatides, egg yolk phospholipids, cardiolipins, glyceroglycolipids, phosphatidylserines, phosphatidylglycerols and aminoethylphosphonolipids and mixtures thereof.

3. The reverse gel of claim 1 wherein said nonfluorinated surfactant is a phospholipid.

4. The reverse gel of claim 3 wherein said phospholipid is natural or modified egg yolk lecithin.

5. The reverse gel of claim 1 wherein the fluorinated continuous phase comprises at least 60% v/v.

6. The reverse gel of claim 1 wherein the fluorinated continuous phase comprises at least 70% v/v.

7. The reverse gel of claim 1 further comprising an additive.

8. The reverse gel of claim 7 wherein said additive is selected from the group consisting of active principles, mineral salts, amino acids, buffering agents, oncotic agents, osmotic agents, nutritive agents, dispersing agents, absorption enhancers, physiological gases, immunoactive principles, organic solvents, polymers, and combinations thereof.

9. The reverse gel of claim 8 wherein the additive is an active principle and said active principle is a therapeutic or diagnostic agent selected from the group consisting of respiratory agents, antibiotics, antivirals, mydriatics, antiglaucomas, anti-inflammatories, antihistaminics, antineoplastics, anesthetics, ophthalmic agents, enzymes, cardiovascular agents, nucleic acids, genetic material, viral vectors, immunoactive agents, imaging agents, immunosuppressive agents, peptides, proteins and gastrointestinal agents.

10. The reverse gel of claim 1 wherein said at least one highly fluorinated organic compound is selected from the group consisting of perfluoroalkanes, fluorinated cyclic compounds, halogenated fluorochemicals, fluorinated alkenes, fluorinated ethers, fluorinated polyethers, fluorinated amines, fluorinated hydrides and derivatives thereof.

11. The reverse gel of claim 1 wherein said partially fluorinated and partially hydrogenated (HC/FC) organic compound comprises a formula selected from the group consisting of C_{N}F_{2N+1}C_{M}H_{2M+1}, C_{N}F_{2N+1}CH₂CH=CHC_{M}H_{2M+1} and C_{N}F_{2N+1}CH=CHC_{M}H_{2M+1} and combinations thereof wherein 2≤n≤12 and 1≤m≤16.

12. The reverse gel of claim 1 further comprising a therapeutically effective amount of at least one physiological gas.

13. The reverse gel according to claim 1 wherein the aqueous phase comprises micelles, vesicles or other colloidal aggregates.

14. A method for forming a fluorinated reverse gel comprising the steps of:
combining at least one partially fluorinated and partially hydrogenated (HC/FC) organic compound with an effective dispersing amount of at least one nonfluorinated surfactant and at least one highly fluorinated organic compound wherein at least 30% of the hydrogen atoms in the corresponding hydrocarbon oil or derivative thereof have been replaced with fluorine atoms to provide a dispersion preparation; and
admixing said dispersion preparation with an aqueous solution to provide a fluorinated reverse gel comprising a disperse aqueous phase of less than 50% v/v.

15. The method of Claim 14, wherein said at least one nonfluorinated surfactant is selected from the group consisting of alcohols, salts of fatty acids, phosphatidylcholines, N-monomethylphosphatidylethanolamines, phosphatidic acids, phosphatidylethanolamines, N,N-dimethylphosphatidylethanolamines, phosphatidyl ethylene glycols, phosphatidylmethanols. phosphatidylethanols, phosphatidylpropanols, phosphatidylbutanols, phosphatidylthioethanols, diphytanoyl phosphatides, egg yolk phospholipids, cardiolipins, glyceroglycolipids, phosphatidylserines, phosphatidylglycerols and aminoethylphosphonolipids and mixtures thereof.

16. The method of claim 14 wherein said nonfluorinated surfactant is a phospholipid.

17. The method of claim 14 further comprising the step of incorporating at least one additive in said fluorinated reverse gel.

18. The method of claim 17 wherein said additive is selected from the group consisting of active principles, mineral salts, amino acids, buffering agents, oncotic agents, osmotic agents, nutritive agents, dispersing agents, absorption enhancers, immunoactive principles, organic solvents, polymers, and combinations thereof.

19. The method of claim 14 wherein said at least one highly fluorinated organic compound is selected from the group consisting of perfluoroalkanes, fluorinated cyclic compounds, halogenated fluorochemicals, fluorinated alkenes, fluorinated ethers, fluorinated polyethers, fluorinated amines, fluorinated hydrides and derivatives thereof.

20. The method of claim 14 wherein said partially fluorinated and partially hydrogenated (HC/FC) organic compound comprises a formula selected from the group consisting of C_{N}F_{2N+1}C_{M}H_{2M+1}, C_{N}F_{2N+1}CH=CHC_{M}H_{2M+1}, C_{N}F_{2N+1}CH=CHC_{M}H_{2M+1}, and combinations thereof wherein 2≤n≤12 and 1≤m≤16.

21. A fluorinated reverse gel formed according to the method of claim 14.

22. A method for the preparation of a fluorinated reverse gel comprising the steps of:
combining a nonfluorinated surfactant with at least one partially fluorinated and partially hydrogenated (HC/FC) organic compound to obtain a homogeneous mixture;
dispersing said homogeneous mixture in at least one highly fluorinated organic compound wherein at least 30% of the hydrogen atoms in the corresponding hydrocarbon oil or derivative thereof have been replaced with fluorine atoms to provide a dispersion preparation; and
adding said dispersion preparation to an aqueous solution to provide a fluorinated reverse gel comprising a dispersed aqueous phase of less than 50% v\v.

23. The method of claim 22 further comprising the step of subjecting said fluorinated reverse gel to a thermal sterilization treatment.

24. The method of Claim 22 wherein said at least one nonfluorinated surfactant is selected from the group consisting of alcohols, salts of fatty acids, phosphatidylcholines, N-monomethyl-phosphatidylethanolamines, phosphatidic acids, phosphatidyl ethanolamines, N,N-dimethyl-phosphatidyl-ethanolamines, phosphatidyl ethylene glycols, phosphatidylmethanols, phosphatidylethanols, phosphatidylpropanols, phosphatidylbutanols, phosphatidylthioethanols, diphytanoyl phosphatides, egg yolk phospholipids, cardiolipins, glycerglycolipids, phosphatidylserines, phosphatidylglycerols and aminoethylphosphonolipids.

25. The method of claim 22 further comprising the step of incorporating at least one active principle in said fluorinated reverse gel.

26. The reverse gel according to any of the claims 7 to 13 for use as a medicament.

27. The reverse gel according to any of the claims 7 to 13 for diagnostic use.

## Patentansprüche

1. Fluoriertes reversibles Gel, das enthält:
- eine kontinuierliche Phase mit wenigstens einer hoch fluorierten organischen Verbindung, in welcher wenigstens 30% der Wasserstoffatome in dem entsprechenden Kohlenwasserstofföl oder in einem Derivat desselben durch Fluoratome ersetzt worden sind und wenigstens eine teilweise fluorierte und teilweise hydrogenierte organische Verbindung (HC/FC) ;
- eine dispergierte wässerige Phase; und
- eine wirksame dispergierende Menge von wenigstens einem nicht fluorierten grenzflächenaktiven Stoff, wobei die dispergierte wässerige Phase weniger als 50 Volumenprozent (Vol.-%) auf das Volumen des reversiblen Gels bezogen ausmacht.

2. Reversibles Gel gemäß Anspruch 1, in welchem der wenigstens eine nicht fluorierte grenzflächenaktive Stoff aus der Gruppe bestehend aus Alkoholen, Salzen von Fettsäuren, Phosphatidylcholinen, N-Monomethylphosphatidylethanolaminen, Phosphatidsäuren, Phosphatidylethanolaminen, N,N-Dimethylphosphatidylethanolaminen, Phosphatidylethylenglykolen, Phosphatidylmethanolen, Phosphatidylethanolen, Phosphatidylpropanolen, Phosphatidylbutanolen, Phosphatidylthioethanolen, Diphytanoylphosphatiden, Phospholipiden aus dem Eigelb, Cardiolipinen, Glyceroglykolipiden, Phosphatidylserinen, Phosphatidylglycerolen und Aminoethylphosphonolipiden und Mischungen derselben ausgewählt wird.

3. Reversibles Gel gemäß Anspruch 1, in welchem der nicht fluorierte grenzflächenaktive Stoff ein Phospholipid ist.

4. Reversibles Gel gemäß Anspruch 3, in welchem das Phospholipid natürlichen Ursprungs ist oder ein modifiziertes Lecithin aus dem Eigelb ist.

5. Reversibles Gel gemäß Anspruch 1, in welchem die fluorierte kontinuierliche Phase wenigstens 60 Vol.-% auf das Volumen des reversiblen Gels bezogen ausmacht.

6. Reversibles Gel gemäß Anspruch 1, in welchem die fluorierte kontinuierliche Phase wenigstens 70 Vol.-% auf das Volumen des reversiblen Gels bezogen ausmacht.

7. Reversibles Gel gemäß Anspruch 1, welches zusätzlich ein Additiv enthält.

8. Reversibles Gel gemäß Anspruch 7, in welchem das Additiv aus der Gruppe bestehend aus aktiven Bestandteilen, Mineralsalzen, Aminsäuren, Puffermitteln, onkozytären Wirkstoffen, osmotischen Wirkstoffen, Nährsubstanzen, dispergierenden Wirkstoffen, Absorptionsaktivatoren, physiologischen Gasen, immunoaktiven Bestandteilen, organischen Lösungsmitteln, Polymeren und Kombinationen derselben ausgewählt wird.

9. Reversibles Gel gemäß Anspruch 8, in welchem das Additiv ein aktiver Bestandteil ist und dieser aktive Bestandteil ein therapeutisches oder diagnostisches Mittel ist, das aus der Gruppe bestehend aus Atmungswirkstoffen, Antibiotika, antiviralen Substanzen, pupillenerweiternden Substanzen, Antiglaukomen, entzündungshemmenden Mitteln, Antihistaminen, antineoplasischen Substanzen, Narkosemitteln, ophtalmischen Wirkstoffen, Enzymen, kardiovaskulären Wirkstoffen, Nukleinsäuren, genetischen Materialien, viralen Vektoren, immunoaktiven Wirkstoffen, bildgebenden Wirkstoffen, immunosuppressiven Wirkstoffen, Peptiden, Proteinen und gastro-intestinalen Wirkstoffen ausgewählt wird.

10. Reversibles Gel gemäß Anspruch 1, in welchem wenigstens eine hoch fluorierte organische Verbindung aus der Gruppe bestehend aus Perfluoralkanen, fluorierten zyklischen Verbindungen, halogenierten Fluorchemikalien, fluorierten Alkenen, fluorierten Ethern, fluorierten Polyethern, fluorierten Aminen, fluorierten Hydriden und Derivaten derselben ausgewählt wird.

11. Reversibles Gel gemäß Anspruch 1, in welchem die teilweise fluorierte und teilweise hydrogenierte organische Verbindung (HC/FC) eine Formel aufweist die aus der Gruppe bestehend aus C_{N}F_{2N+1}C_{M}H_{2M+1}, C_{N}F_{2N+1}CH₂CH=CHC_{M}H_{2M+1} und C_{N}F_{2N+1}CH=CHC_{M}H_{2M+1} und aus Kombinationen derselben, in welchen 2 ≤ N ≤ 12 und 1 ≤ M ≤ 16, ausgewählt wird.

12. Reversibles Gel gemäß Anspruch 1, welches zusätzlich eine therapeutisch wirksame Menge von wenigstens einem physiologischen Gas enthält.

13. Reversibles Gel gemäß Anspruch 1, in welchem die wässerige Phase Mizellen, Vesikel und andere kolloïdale Aggregate enthält.

14. Verfahren für die Bildung eines fluorierten reversiblen Gels, das die folgenden Schritte umfasst:
- die Kombination von wenigstens einer teilweise fluorierten und teilweise hydrogenierten organischen Verbindung (HC/FC) mit einer wirksamen dispergierenden Menge von wenigstens einem nicht fluorierten grenzflächenaktiven Stoff und wenigstens einer hoch fluorierten organischen Verbindung in welcher wenigstens 30% der Wasserstoffatome in dem entsprechenden Kohlenwasserstofföl oder Derivat desselben durch Fluoratome ersetzt worden sind, um eine Zubereitung einer Dispersion zu ergeben; und
- das Mischen der Zubereitung der Dispersion mit einer wässerigen Lösung, um ein fluoriertes reversibles Gel zu ergeben, das eine dispergierte wässerige Phase von weniger als 50 Vol.-% auf das Volumen des reversiblen Gels bezogen enthält.

15. Verfahren gemäß Anspruch 14, in welchem wenigstens ein nicht fluorierter grenzflächenaktive Stoff aus der Gruppe bestehend aus Alkoholen, Salzen von Fettsäuren, Phosphatidylcholinen, N-Monomethylphosphatidylethanolaminen, Phosphatidsäuren, Phosphatidylethanolaminen, N,N-Dimethylphosphatidylethanolaminen, Phosphatidylethylenglykolen, Phosphatidylmethanolen, Phosphatidylethanolen, Phosphatidylpropanolen, Phosphatidylbutanolen, Phosphatidylthioethanolen, Diphytanoylphosphatiden, Phospholipiden aus dem Eigelb, Cardiolipinen, Glyceroglykolipiden, Phosphatidylserinen, Phosphatidylglycerolen und Aminoethylphosphonolipiden und den Mischungen derselben ausgewählt wird.

16. Verfahren gemäß Anspruch 14, in welchem der nicht fluorierte grenzflächenaktive Stoff ein Phospholipid ist.

17. Verfahren gemäß Anspruch 14, das ferner den Schritt der Einverleibung von wenigstens einem Additiv in das fluorierte reversible Gel aufweist.

18. Verfahren gemäß Anspruch 17, in welchem das Additiv aus der Gruppe bestehend aus aktiven Bestandteilen, Mineralsalzen, Aminsäuren, Puffermitteln, onkozytären Wirkstoffen, osmotischen Wirkstoffen, Nährsubstanzen, dispergierenden Wirkstoffen, Absorptionsaktivatoren, immunoaktiven Bestandteilen, organischen Lösungsmitteln, Polymeren und Kombinationen derselben ausgewählt wird.

19. Verfahren gemäß Anspruch 14, in welchem wenigstens eine hoch fluorierte organische Verbindung aus der Gruppe bestehend aus Perfluoralkanen, fluorierten zyklischen Verbindungen, halogenierten Fluorchemikalien, fluorierten Alkenen, fluorierten Ethern, fluorierten Polyethern, fluorierten Aminen, fluorierten Hydriden und Derivaten derselben ausgewählt wird.

20. Verfahren gemäß Anspruch 14, in welchem die teilweise fluorierte und teilweise hydrogenierte organische Verbindung (HC/FC) eine Formel aufweist, die aus der Gruppe bestehend aus CNF2N+1CMH2M+1, C_{N}F_{2N}+1CH₂CH = CHC_{M}H_{2M}+1 und C_{N}F_{2N+1}CH = CHC_{M}H_{2M+1} und aus Kombinationen derselben, in welchen 2 ≤ N ≤ 12 und 1 ≤ M ≤ 16, ausgewählt wird.

21. Reversibles fluoriertes Gel, das nach dem Verfahren nach Anspruch 14 erzeugt worden ist.

22. Verfahren für die Zubereitung eines fluorierten reversiblen Gels, das die folgenden Schritte umfasst:
- die Kombination eines nicht fluorierten grenzflächenaktiven Stoffs mit wenigstens einer teilweise fluorierten und teilweise hydrogenierten organischen Verbindung (HC/FC), um ein homogenes Gemisch zu erzielen;
- das Dispergieren des homogenen Gemischs in wenigstens einer hoch fluorierten organischen Verbindung, in welcher wenigstens 30% der Wasserstoffatome in dem entsprechenden Kohlenwasserstofföl oder Derivat desselben durch Fluoratome ersetzt worden sind, um eine Zubereitung einer Dispersion zu ergeben; und
- die Zugabe der Zubereitung der Dispersion zu einer wässerigen Lösung, um ein fluoriertes reversibles Gel zu ergeben, das eine dispergierte wässerige Phase von weniger als 50 Vol.-% auf das Volumen des reversiblen Gels bezogen enthält.

23. Verfahren gemäß Anspruch 22, das ferner den Schritt umfasst, der darin besteht das fluorierte reversible Gel der Behandlung einer thermischen Sterilisation zu unterziehen.

24. Verfahren gemäß Anspruch 22, in welchem der wenigstens eine nicht fluorierte grenzflächenaktive Stoff aus der Gruppe bestehend aus Alkoholen, Salzen von Fettsäuren, Phosphatidylcholinen, N-Monomethylphosphatidylethanolaminen, Phosphatidsäuren, Phosphatidylethanolaminen, N,N-Dimethylphosphatidylethanolaminen, Phosphatidylethylenglykolen, Phosphatidylmethanolen, Phosphatidylethanolen, Phosphatidylpropanolen, Phosphatidylbutanolen, Phosphatidylthioethanolen, Diphytanoylphosphatiden, Phospholipiden aus dem Eigelb, Cardiolipinen, Glyceroglykolipiden, Phosphatidylserinen, Phosphatidylglycerolen und Aminoethylphosphonolipiden ausgewåhlt wird.

25. Verfahren gemäß Anspruch 22, das ferner den Schritt der Einverleibung von wenigstens einem aktiven Wirkstoff in das fluorierte reversible Gel aufweist.

26. Reversibles Gel gemäß irgendeinem der Ansprüche 7 bis 13 für die Anwendung als Arzneimittel.

27. Reversibles Gel gemäß irgendeinem der Ansprüche 7 bis 13 für die diagnostische Anwendung.

## Revendications

1. Gel inverse fluoré comprenant:
- une phase continue comprenant au moins un composé organique hautement fluoré, dans lequel au moins 30% des atomes d'hydrogène dans l'huile hydrocarbure correspondante ou un dérivé de celle-ci ont été remplacés par des atomes de fluor, et au moins un composé organique partiellement fluoré et partiellement hydrogéné (HC/FC);
- une phase aqueuse dispersée; et
- une quantité dispersante efficace d'au moins un tensioactif non fluoré, dans lequel ladite phase aqueuse dispersée constitue moins de 50% volume pour volume dudit gel inverse.

2. Gel inverse selon la revendication 1, dans lequel ledit au moins un tensioactif non fluoré est choisi dans le groupe constitué d'alcools, de sels d'acides gras, de phosphatidylcholines, de N-monométhylphosphatidyléthanolamines, d'acides phosphatidiques, de phosphatidyléthanolamines, de N,N-diméthylphosphatidyléthanolamines, de phosphatidyléthylèneglycols, de phosphatidylméthanols, de phosphatidyléthanols, de phosphatidylpropanols, de phosphatidylbutanols, de phosphatidylthioéthanols, de diphytanoylphosphatides, de phospholipides de jaune d'oeuf, de cardiolipines, de glycéroglycolipides, de phosphatidylsérines, de phosphatidylglycérols et d'aminoéthylphosphonolipides et de mélanges de ceux-ci.

3. Gel inverse selon la revendication 1, dans lequel ledit tensioactif non fluoré est un phospholipide.

4. Gel inverse selon la revendication 3, dans lequel ledit phospholipide est de la lécithine de jaune d'oeuf modifiée ou naturelle.

5. Gel inverse selon la revendication 1, dans lequel la phase continue fluorée comprend au moins 60% volume pour volume.

6. Gel inverse selon la revendication 1, dans lequel la phase continue fluorée comprend au moins 70% volume pour volume.

7. Gel inverse selon la revendication 1, comprenant en outre un additif.

8. Gel inverse selon la revendication 7, dans lequel ledit additif est choisi dans le groupe constitué de principes actifs, de sels minéraux, d'acides aminés, d'agents tampons, d'agents oncotiques, d'agents osmotiques, d'agents nutritifs, d'agents dispersants, d'activateurs d'absorption, de gaz physiologiques, de principes immunoactifs, de solvants organiques, de polymères et de combinaisons de ceux-ci.

9. Gel inverse selon la revendication 8, dans lequel l'additif est un principe actif et ledit principe actif est un agent thérapeutique ou diagnostique choisi dans le groupe constitué d'agents respiratoires, d'antibiotiques, d'antiviraux, de mydriatiques, d'antiglaucomes, d'anti-inflammatoires, d'antihistaminiques, d'antinéoplasiques, d'anesthésiques, d'agents ophtalmiques, d'enzymes, d'agents cardiovasculaires, d'acides nucléiques, de matériel génétique, de vecteurs viraux, d'agents immunoactifs, d'agents d'imagerie, d'agents immunosuppresseurs, de peptides, de protéines et d'agents gastro-intestinaux.

10. Gel inverse selon la revendication 1, dans lequel ledit au moins un composé organique hautement fluoré est choisi dans le groupe constitué de perfluoroalcanes, de composés cycliques fluorés, de composés fluorés halogénés, d'alcènes fluorés, d'éthers fluorés, de polyéthers fluorés, d'aminés fluorées, d'hydrures fluorés et de dérivés de ceux-ci.

11. Gel inverse selon la revendication 1, dans lequel ledit composé organique partiellement fluoré et partiellement hydrogéné (HC/FC) comprend une formule choisie dans le groupe constitué de CₙF₂ₙ+1CₘH₂ₘ+1, de CₙF₂ₙ+1CH₂CH = CHCₘH₂ₘ+1 et de CₙF₂ₙ+1CH = CHCₘH₂ₘ+1 et de combinaisons de celles-ci, dans lesquelles 2 ≤ n ≤ 12 et 1 ≤ m ≤ 16.

12. Gel inverse selon la revendication 1, comprenant en outre une quantité thérapeutiquement efficace d'au moins un gaz physiologique.

13. Gel inverse selon la revendication 1, dans lequel la phase aqueuse comprend des micelles, des vésicules ou d'autres agrégats colloïdaux.

14. Méthode de formation d'un gel inverse fluoré comprenant les étapes de:
- combinaison d'au moins un composé organique partiellement fluoré et partiellement hydrogéné (HC/FC) avec une quantité dispersante efficace d'au moins un tensioactif non fluoré et d'au moins un composé organique hautement fluoré dans lequel au moins 30% des atomes d'hydrogène dans l'huile hydrocarbure correspondante ou un dérivé de celle-ci ont été remplacés par des atomes de fluor pour donner une préparation de dispersion; et
- mélange de ladite préparation de dispersion avec une solution aqueuse pour donner un gel inverse fluoré comprenant une phase aqueuse dispersée de moins de 50% volume pour volume.

15. Méthode selon la revendication 14, dans laquelle ledit au moins un tensioactif non fluoré est choisi dans le groupe constitué d'alcools, de sels d'acides gras, de phosphatidylcholines, de N-monométhylphosphatidyléthanolamines, d'acides phosphatidiques, de phosphatidyléthanolamines, de N,N-diméthylphosphatidyléthanolamines, de phosphatidyléthylèneglycols, de phosphatidylméthanols, de phosphatidyléthanols, de phosphatidylpropanols, de phosphatidylbutanols, de phosphatidylthioéthanols, de diphytanoylphosphatides, de phospholipides de jaune d'oeuf, de cardiolipines, de glycéroglycolipides, de phosphatidylsérines, de phosphatidylglycérols et d'aminoéthylphosphonolipides et de mélanges de ceux-ci.

16. Méthode selon la revendication 14, dans laquelle ledit tensioactif non fluoré est un phospholipide.

17. Méthode selon la revendication 14, comprenant en outre l'étape d'incorporation d'au moins un additif dans ledit gel inverse fluoré.

18. Méthode selon la revendication 17, dans laquelle ledit additif est choisi dans le groupe constitué de principes actifs, de sels minéraux, d'acides aminés, d'agents tampons, d'agents oncotiques, d'agents osmotiques, d'agents nutritifs, d'agents dispersants, d'activateurs d'absorption, de principes immunoactifs, de solvants organiques, de polymères et de combinaisons de ceux-ci.

19. Méthode selon la revendication 14, dans laquelle ledit au moins un composé organique hautement fluoré est choisi dans le groupe constitué de perfluoroalcanes, de composés cycliques fluorés, de composés fluorés halogénés, d'alcènes fluorés, d'éthers fluorés, de polyéthers fluorés, d'amines fluorées, d'hydrures fluorés et de dérivés de ceux-ci.

20. Méthode selon la revendication 14, dans laquelle ledit composé organique partiellement fluoré et partiellement hydrogéné (HC/FC) comprend une formule choisie dans le groupe constitué de CₙF₂ₙ+1CₘH₂ₘ+1, de CₙF₂ₙ+1CH = CHCₘH₂ₘ+1 et de CₙF₂ₙ+1CH = CHCₘH₂ₘ₊₁ et de combinaisons de celles-ci, dans lesquelles 2 ≤ n ≤ 12 et 1 ≤ m ≤ 16.

21. Gel inverse fluoré formé selon la méthode de la revendication 14.

22. Méthode pour la préparation d'un gel inverse fluoré comprenant les étapes de:
- combinaison d'un tensioactif non fluoré avec au moins un composé organique partiellement fluoré et partiellement hydrogéné (HC/FC) pour obtenir un mélange homogène;
- dispersion dudit mélange homogène dans au moins un composé organique hautement fluoré dans lequel au moins 30% des atomes d'hydrogène dans l'huile hydrocarbure correspondante ou un dérivé de celle-ci ont été remplacés par des atomes de fluor pour donner une préparation de dispersion; et
- ajout de ladite préparation de dispersion à une solution aqueuse pour donner un gel inverse fluoré comprenant une phase aqueuse dispersée de moins de 50% volume pour volume.

23. Méthode selon la revendication 22, comprenant en outre l'étape consistant à soumettre ledit gel inverse fluoré à un traitement de stérilisation thermique.

24. Méthode selon la revendication 22, dans laquelle ledit au moins un tensioactif non fluoré est choisi dans le groupe constitué d'alcools, de sels d'acides gras, de phosphatidylcholines, de N-monométhylphosphatidyléthanolamines, d'acides phosphatidiques, de phosphatidyléthanolamines, de N,N-diméthylphosphatidyléthanolamines, de phosphatidyléthylèneglycols, de phosphatidylméthanols, de phosphatidyléthanols, de phosphatidylpropanols, de phosphatidylbutanols, de phosphatidylthioéthanols, de diphytanoylphosphatides, de phospholipides de jaune d'oeuf, de cardiolipines, de glycéroglycolipides, de phosphatidylsérines, de phosphatidylglycérols et d'aminoéthylphosphonolipides.

25. Méthode selon la revendication 22, comprenant en outre l'étape d'incorporation d'au moins un principe actif dans ledit gel inverse fluoré.

26. Gel inverse selon l'une quelconque des revendications 7 à 13, pour une utilisation en tant que médicament.

27. Gel inverse selon l'une quelconque des revendications 7 à 13, pour une utilisation diagnostique.
